# EUROPEAN PATENT SPECIFICATION

(11) **EP 2 545 183 B1**
(45) Date of publication and mention of the grant of the patent: **19.04.2017**
(21) Application number: 11754012.0
(22) Date of filing: 09.03.2011
(51) Int. Cl.: C12N 15/10, C12N 15/66, C12N 9/22, C12P 19/34, C40B 40/08, C40B 50/06

(54) **PRODUCTION OF SINGLE-STRANDED CIRCULAR NUCLEIC ACID**
HERSTELLUNG EINER EINSTRÄNGIGEN ZIRKULÄREN NUKLEINSÄURE
PRODUCTION D'ACIDE NUCLÉIQUE CIRCULAIRE MONOCATÉNAIRE

(30) Priority: 09.03.2011 US 201113044221; 10.03.2010 US 312332 P
(43) Date of publication of application: 16.01.2013
(73) Proprietor: Ibis Biosciences, Inc., Carlsbad, CA 92008 (US)
(72) Inventor: ESHOO, Mark W., Solana Beach California 92075 (US); PICURI, John, Carlsbad California 92009 (US)
(74) Representative: Chapman, Desmond Mark
(86) International application number: PCT/US2011/027753
(87) International publication number: WO 2011/112718

(56) References cited:
- WO-A1-2010/048605
- WO-A1-2010/094040
- WO-A1-2012/092260
- WO-A2-2009/120372
- US-A1- 2008 305 535
- US-A1- 2010 120 098
- CARUCCIO NICHOLAS: "Preparation of next-generation sequencing libraries using Nextera(TM) technology: simultaneous DNA fragmentation and adaptor tagging by in vitro transposition", METHODS IN MOLECULAR BIOLOGY, HUMANA PRESS, INC, US, vol. 733, 1 January 2011 (2011-01-01), pages 241-255, XP009157814, ISSN: 1940-6029, DOI: 10.1007/978-1-61779-089-8_17 [retrieved on 2011-01-01]
- SYED ET AL.: 'Optimized library preparation method for next-generation sequencing' NATURE METHODS October 2009, page I, II, XP002672202
- BHASIN ET AL.: 'Hairpin Formation in Tn5 Transposition' THE JOURNAL OF BIOLOGICAL CHEMISTRY vol. 274, no. 52, 24 December 1999, pages 37021 - 37029, XP002213929

## Description

### FIELD OF THE INVENTION

The present invention relates to the field of analysis of nucleic acids and more particularly to methods for producing single-stranded circular nucleic acid for improving the efficiency of amplification and sequencing technologies.

### BACKGROUND OF THE INVENTION

Single-stranded circular DNA has been found to be useful in many different areas of biotechnology. One important use is as a substrate for rolling circle DNA replication. In this procedure, a single-stranded circle of DNA is mixed with a short strand of single-stranded complementary primer DNA and the two separate strands are allowed to anneal. After addition of a DNA polymerase, such as the Klenow fragment, the intact circle is used as a template by the enzyme and then replicated from the 3'-end of the primer strand. After the enzyme has gone around the circular template, it encounters the 5'-end of the primer, which is then displaced from the template strand so that the enzyme continues to move around the circular template while a long, unbroken single strand of DNA is generated. Such single strand has been referred to as single-stranded concatenated DNA. Single-stranded circular products are ideally suited for use as a substrate in such processes. Such products can ultimately yield single-strand concatenated DNA having numerous different sequential segments that can act as probes, detection sites or restriction sites for further processing.

Libraries of single-strand circular DNA are also useful for rapid DNA sequencing methods. Single molecule real time (SMRT) DNA sequencing technologies have the capability to re-sequence a single segment of double-stranded DNA repeatedly by using a single-strand circular DNA molecule as a template. Pyrosequencing is another rapid sequencing method which would benefit from the use of libraries of single-strand circular DNA (SMRT and pyrosequencing methods are described, for example in PCT publication WO2009120372).

WO 2009/120372 teaches compositions and methods for nucleic acid sequencing which involve template constructs that comprise double stranded portions in partially or completely contiguous constructs, to provide for redundant sequence determination through one or both of sequencing sense and antisense strands.

WO 2010/048605 teaches methods, compositions and kits for generating 5'- and 3'-tagged DNA fragments that comprise using a transposase and a transposon end for generating extensive fragmentation and 5'-tagging of double-stranded target DNA in vitro.

Syed et al. (Nature Methods, October 2009, page i) describes a transposon-based method for preparing fragmented and tagged libraries for next-generation sequencing.

Bhasin et al. (Journal of Biological Chemistry, December 1999, 274:52, pages 37021-37029) demonstrates that the initial chemical steps in Tn5 transposition result in blunt end cleavage of the transposon from the donor DNA via a hairpin intermediate.

The present invention relates to methods for producing single-stranded circular nucleic acid.

### SUMMARY OF THE INVENTION

An object of the invention is to provide a rapid and unbiased method for generating single-stranded circular nucleic acids for use as templates in applications such as nucleic acid sequencing and rolling circle amplification of nucleic acids.

A further object of the invention is to provide a method for generating single-stranded circular nucleic acid molecules using simplified processes which are amenable to adaptation within microfluidics devices.

The invention provides a method for generating single stranded circular nucleic acid from a sample of target nucleic acid, said method comprising: (a) forming a complex comprising a transposase and a plurality of hairpin polynucleotides, each of said hairpin polynucleotides having a duplex region comprising a transposase recognition sequence; (b) mixing said complex with said target nucleic acid, thereby fragmenting said target nucleic acid and ligating said hairpin polynucleotides to said target nucleic acid, to form hairpin-linked nucleic acid fragments, each hairpin-linked nucleic acid fragment having a duplex target nucleic acid fragment region and a nucleobase segment gap between each target nucleic acid fragment region and its corresponding hairpin polynucleotide; (c) contacting said hairpin-linked fragments with a ligase, thereby ligating the duplex target nucleic acid fragment regions of said hairpin-linked fragments together to form single-stranded circular nucleic acid comprising a pair of opposing loops and an intervening duplex region, said duplex region comprising a pair of nucleobase segment gaps; and (d) contacting said single-single stranded circular nucleic acid of step (c) with a polymerase and nucleotide triphosphates, thereby filling said nucleobase segment gaps.

The invention also provides a method for preparing a library of single-stranded circular nucleic acid which represents a genome of a virus or organism, said method comprising: (a) forming a complex comprising a transposase and a plurality of hairpin polynucleotides, each of said hairpin polynucleotides having a duplex region comprising a transposase recognition sequence; (b) mixing said complex with nucleic acid representing said genome, thereby fragmenting said nucleic acid and ligating said hairpin polynucleotides to said nucleic acid, to form hairpin-linked nucleic acid fragments, each hairpin-linked nucleic acid fragment having a duplex target nucleic acid fragment region and a nucleobase segment gap between each target nucleic acid fragment region and its corresponding hairpin polynucleotide; (c) contacting said hairpin-linked fragments with a ligase, thereby ligating the duplex target nucleic acid fragment regions of said hairpin-linked fragments together to form single-stranded circular nucleic acid comprising a pair of opposing loops and an intervening duplex region, said duplex region comprising a pair of nucleobase segment gaps; and (d) contacting said single-single stranded circular nucleic acid of step (c) with a polymerase and nucleotide triphosphates, thereby filling said nucleobase segment gaps.

The methods disclosed herein employ hairpin polynucleotides and a transposase to fragment nucleic acid segments and ligate hairpin polynucleotides to the fragments. A polymerase is then used to fill in the nucleobase segment gaps formed by the fragmentation process. These methods eliminate the need for fragmenting genomic nucleic acid and waiting for slow ligation reactions. The random nucleic acid integration reactions catalyzed by certain transposases provide unbiased generation of fragments.

A method is disclosed herein for generating single stranded circular nucleic acid from a sample of target nucleic acid. A complex comprising a transposase and a plurality of hairpin polynucleotides is formed with each of the hairpin polynucleotides having a duplex region comprising a transposase recognition sequence. The complex is mixed with the target nucleic acid, thereby fragmenting the target nucleic acid and ligating the hairpin polynucleotides to the target nucleic acid to form hairpin-linked nucleic acid fragments, each having a nucleobase segment gap between each fragment and its corresponding hairpin polynucleotide. The hairpin-linked fragments are contacted with a ligase, thereby ligating the hairpin-linked fragments together to form single-stranded circular nucleic acid comprising a pair of opposing loops and an intervening duplex region comprising a pair of nucleobase segment gaps. The single-single stranded circular nucleic acid is then contacted with a polymerase and nucleotide triphosphates, thereby filling the nucleobase segment gaps.

In another aspect, a method is disclosed herein for preparing a library of single-stranded circular nucleic acid which represents a genome of a virus or organism. A complex is formed comprising a transposase and a plurality of hairpin polynucleotides, each having a duplex region comprising a transposase recognition sequence. The complex is mixed with nucleic acid representing the genome, thereby fragmenting the nucleic acid and ligating the hairpin polynucleotides to the nucleic acid to form hairpin-linked nucleic acid fragments, each having a nucleobase segment gap between each fragment and its corresponding hairpin polynucleotide. The hairpin-linked fragments are contacted with a ligase, thereby ligating the hairpin-linked fragments together to form a single-stranded circular nucleic acid comprising a pair of opposing loops and an intervening duplex region which comprises a pair of nucleobase segment gaps. The single-single stranded circular nucleic acid is contacted with a polymerase and nucleotide triphosphates, thereby filling the nucleobase segment gaps.

In another aspect, a kit is disclosed herein for preparing single-stranded circular DNA. The kit comprises a hairpin polynucleotide comprising a transposase recognition sequence, a transposase, a polymerase and a ligase.

In another aspect, a system is disclosed herein for generating single-stranded circular nucleic acid from a target nucleic acid. The system comprises a first reaction chamber provided with a first set of reaction buffer components configured for formation of a complex between a transposase and a plurality of hairpin polynucleotides, each of the hairpin polynucleotides having a duplex region comprising a transposase recognition sequence. The system comprises a second reaction chamber provided with a second set of reaction buffer components configured for fragmenting the nucleic acid and ligating the hairpin polynucleotides to the nucleic acid, to form hairpin-linked nucleic acid fragments, each having a nucleobase segment gap between each fragment and its corresponding hairpin polynucleotide. The system further comprises a third reaction chamber provided with a third set of buffer components which is compatible with ligase, polymerase and nucleotide triphosphates. The system further comprises a liquid handler configured to transfer aliquots of solutions from the first reaction chamber to the second reaction chamber and from the second reaction chamber to the third reaction chamber.

In some embodiments of the system, a purification module is provided for purifying the single-stranded circular nucleic acid. The purification module is in liquid handling communication with the third chamber.

In some embodiments, the system is provided within a microfluidics chip.

In some embodiments, the methods further include the step of heating the single stranded circular nucleic acid to denature the intervening duplex region. Other method steps may further include mixing the hairpin-linked fragments with a kinase before or during the step of filling in the nucleobase segment gaps to phosphorylate any non-phosphorylated 5'-ends.

In certain embodiments of the methods and kits, the transposase is MuA transposase or Tn5 transposase. In embodiments where the transposase is Tn5 transposase, the transposase recognition sequence employed may be the 19-base pair mosaic end sequence of the Tn5 transposon. In other embodiments where the transposase is MuA transposase, the transposase recognition sequence used may be the R1 and/or the R2 region of the MuA transposon.

In some embodiments, the transposase catalyzes random integration of the hairpin polynucleotide into the target nucleic acid.

In some embodiments, the polymerase lacks 5'-3' exonuclease activity and/or strand-displacement activity.

In some embodiments, the polymerase is T4 polymerase or T7 polymerase and the ligase is T4 ligase or *E. coli* ligase.

In some embodiments, the hairpin polynucleotides comprise sequencing tags.

In certain embodiments of the kits disclosed herein, instructions are provided for performing a series of reactions to produce single-stranded circular nucleic acid. The kit may be used to produce a library of single-stranded circular nucleic acids.

The single-stranded circular nucleic acid produced according to the method described herein can be used as a template for amplification or sequencing.

The library of single-stranded circular nucleic acids produced according to the method described herein can also be used for amplification or sequencing.

### BRIEF DESCRIPTION OF THE DRAWINGS

The foregoing summary and detailed description is better understood when read in conjunction with the accompanying drawings which are included by way of example and not by way of limitation.
FIG. 1 is a schematic representation of the process of obtaining single-stranded circular nucleic acid according to one embodiment.
FIG. 2 is a schematic representation of a system for producing single-stranded circular DNA according to one embodiment.

### DETAILED DESCRIPTION OF EMBODIMENTS

### I. General

The next generation of rapid nucleic acid sequencing technologies, such as single molecule real-time sequencing have the capability to re-sequence a single segment of double-stranded DNA repeatedly by using a single-stranded circular DNA molecule as a template (see for example, WO2009120372, Caruccio et al., Nextera™ Technology for NGS DNA Library Preparation: Simultaneous Fragmentation and Tagging by In Vitro Transposition Epicentre Forum 2009, 16-3, 4-6). Currently, DNA libraries comprising single-stranded circular DNA are produced using physical methods for fragmentation of DNA such as sonication and nebulization among others. This step is followed by selection of fragments of appropriate length and subjecting them to enzymatic processing to prepare the sample for sequencing. These physical methods require large amounts of DNA, on the order of microgram quantities, and are generally inefficient.

Methods are needed to improve the process for preparing single-stranded circular nucleic acids and libraries thereof in order to speed the "front-end" processing work required to take advantage of rapid sequencing technologies.

Transposons are found in all the biological kingdoms, and some perform specialized functions. For example, the genome of bacteriophage Mu includes a transposon that uses transposition both to integrate into the DNA of a new host cell and to replicate before lysis. Like most DNA rearrangements, transposition is a complex, multistep process, requiring numerous DNA sequence elements. Studies of bacteriophage Mu have been central to our understanding of both the fundamental mechanisms and the complexities of DNA transposition.

Phage Mu encodes the MuA transposase which transfers the Mu genome from one DNA location (the transposition donor) to a new location (the transposition target). During transposition, transposase performs two principle reactions: DNA cleavage and DNA strand transfer. During cleavage, the donor DNA is nicked twice, once at each 3'-end of the Mu genome. During strand transfer, the cleaved transposon ends are inserted into neighboring sites on the two target strands.

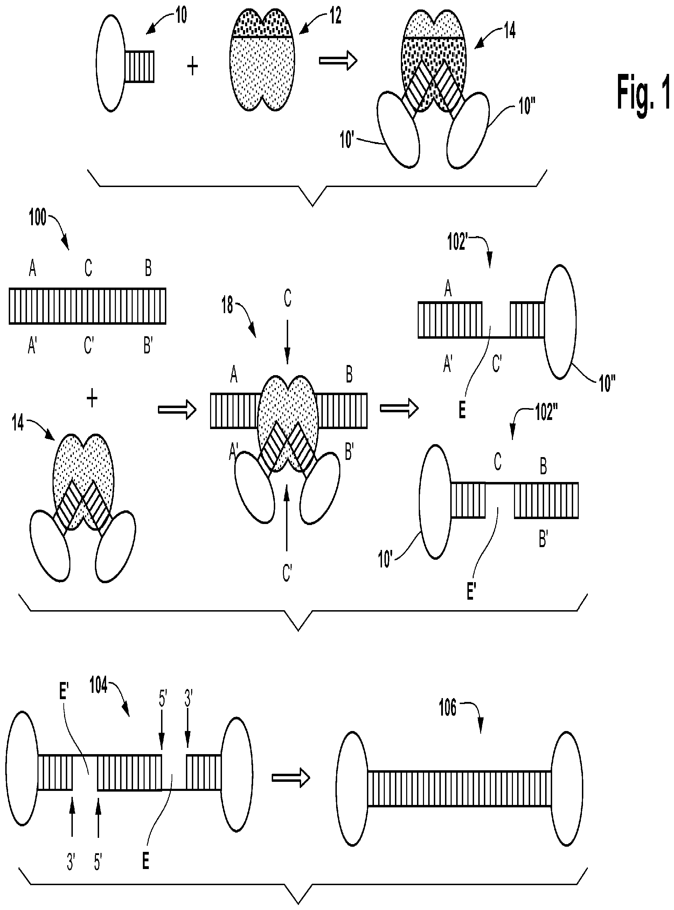

Little or no specific sequence information is needed on the target DNA, but the Mu DNA provides many sequence cues for transposition. For example, the last two nucleotides at either 3' end of the Mu DNA, the cleavage sites, have the sequence 5'-CA. Also near each end of the Mu DNA are three recognition sites, distinct from the cleavage sites, which share a 22-base pair consensus sequence. The recognition sites are referred to as R1, R2, and R3 on the right end and L1, L2, and L3 on the left end (Fig. 1). The recognition sites are bound specifically by the N-terminal domain of MuA, whereas the cleavage sites must be engaged by the protein's active site, contained in a different region of the protein. Both the recognition sequences and the 5'-CA cleavage sequences are required for transposition (Goldhaber-Gordon et al., J. Biol. Chem. 2002, 277, 7694-7702).

Another example of a transposase is Tn5 transposase, which has been adapted as a molecular biology reagent (EZ-Tn5™ Transposase) by Epicentre Biotechnologies Inc. (http://www.epibio.com). Applications of this reagent include *in vitro* insertion of an EZ-Tn5 Transposon into DNA cloned in vectors, such as plasmids, fosmids, cosmids, or BACs as well as in vitro insertion into linear DNA. The reagent may also be used for preparation of EZ-Tn5 transposomes for *in vivo* transposition following electroporation into living cells.

The EZ-Tn5™ Transposase reagent is a hyperactive form of Tn5 transposase. The highly purified, single-subunit enzyme can be used to randomly insert (transpose or "hop") any EZ-Tn5 Transposon into any target DNA in vitro with an efficiency up to 106 insertion clones per standard reaction. When incubated with an EZ-Tn5 Transposon™ in the absence of Mg²⁺, a stable EZ-Tn5 Transposome™ complex is formed. The transposome is so stable that it can be electroporated into living cells. Once in the cell, the transposome is activated by intracellular Mg²⁺ and the EZ-Tn5 transposon component is randomly inserted into the host's genomic DNA.

A typical EZ-Tn5 transposition reaction requires four components: (1) the EZ-Tn5 Transposase™; (2) an EZ-Tn5 transposon; (3) a target DNA; and (4) the presence of Mg²⁺. The highly random insertion of an EZ-Tn5 transposon into the target DNA proceeds by a cut-and-paste mechanism catalyzed by the EZ-Tn5 Transposase™, and results in a 9-bp duplication of target DNA sequence immediately adjacent to both ends of the transposon.

### II. Definitions

It is to be understood that the terminology used herein is for the purpose of describing particular embodiments only, and is not intended to be limiting. Further, unless defined otherwise, all technical and scientific terms used herein have the same meaning as commonly understood by one of ordinary skill in the art to which this invention pertains. In describing and claiming the present invention, the following terminology and grammatical variants will be used in accordance with the definitions set forth below.

A "hairpin polynucleotide", as used herein, is a single stranded polynucleotide having two regions which are sufficiently complementary that they hybridize to each other. Preferably, the two regions are completely complementary. As shown in Figure IA, a hairpin polynucleotide is composed of two portions: a "stem" and a "cap" or "loop" region. In the "stem" region, complementary bases are paired in a typical antiparallel duplex. If the two complementary portions of the polynucleotide are separated by bases which do not form a complementary structure, then the bases form a single stranded loop off the end of the stem. A single stranded polynucleotide in which the two complementary regions comprise the entire molecule is said to be a "perfect hairpin." A perfect hairpin has a "cap" portion that is generally about four bases in length. As used herein, the term "loop" also encompasses a cap unless specified otherwise. A loop can contain from 3 to about three thousand bases. Also included in the definition of hairpin polynucleotide is a circular polynucleotide having two regions that are sufficiently complementary that they hybridize to each other. In this case there is a loop portion at both ends of the double stranded stem portion.

The nucleotides in the double stranded portion of the hairpin generally outnumber the nucleotides in the loop portion. For example, the double stranded portion of the hairpin can comprise about 60% or more of the nucleotides. In some applications, the double stranded portion of the hairpin can comprise more than about 90% of the nucleotides and, at times, more than about 99% of the nucleotides.

A hairpin polynucleotide that is not circular has a 3' end and a 5' end. The portion in the stem of the hairpin with the 3' end is referred to as the "3' portion." The portion in the stem of the hairpin with the 5' end is referred to as the "5' portion."

As used herein, the term "transposase" refers to an enzyme that binds to the ends of a transposon and catalyzes the movement of the transposon to another part of the genome by a cut and paste mechanism or a replicative transposition mechanism.

As used herein, the term "transposon" refers to a sequence of nucleic acid which can move around to different positions within the genome of a single cell in a process known as transposition. In the process, they can cause mutations and change the amount of DNA in the genome. Transposons were also once called jumping genes, and are examples of mobile genetic elements. There are several mobile genetic elements and they can be grouped based on their mechanism of transposition. Class I mobile genetic elements, or retrotransposons, copy themselves by first being transcribed to RNA, then reverse transcribed back to DNA by reverse transcriptase, and then being inserted at another position in the genome. Class II mobile genetic elements move directly from one position to another using a transposase to "cut and paste" them within the genome.

As used herein, the term "ligase" refers to an enzyme which catalyzes the joining of two large molecules by forming a new chemical bond, usually with accompanying hydrolysis of a small chemical group pendant to one of the larger molecules. For example, DNA ligase is an enzyme commonly used in molecular biology laboratories to join together DNA fragments. Other common names for ligases include synthetases, because they are used to synthesize new molecules.

As used herein, the term "polymerase" refers to an enzyme whose central function is associated with polymers of nucleic acids such as RNA and DNA. The primary function of a polymerase is the polymerization of new DNA or RNA against an existing DNA or RNA template in the processes of replication and transcription. In association with a cluster of other enzymes and proteins, they use free nucleotides (usually in the form of nucleotide triphosphates) in the solvent, and catalyze the synthesis of a polynucleotide sequence against a nucleotide template strand using base-pairing interactions.

As used herein, the terms "processivity" and "processive" refer to a measure of the average number of nucleotides added by a DNA polymerase enzyme per association/disassociation with the template. DNA polymerases associated with DNA replication tend to be highly processive, while those associated with DNA repair tend to have low processivity. Multiple DNA polymerases have specialized roles in the DNA replication process. For example, in *E. coli*, which replicates its entire genome from a single replication fork, the polymerase DNA Pol III is the enzyme primarily responsible for DNA replication and forms a replication complex with extremely high processivity. The related DNA Pol I has exonuclease activity and serves to degrade the RNA primers used to initiate DNA synthesis. Pol I then synthesizes the short DNA fragments that were formerly hybridized to the RNA fragment. Thus Pol I is much less processive than Pol III because its primary function in DNA replication is to create many short DNA regions rather than a few very long regions.

As used herein, the term "kinase" refers to an enzyme that transfers phosphate groups from high-energy donor molecules, such as ATP, to specific substrates. The process is referred to as phosphorylation. An alternative to the term "kinase" is "phosphotransferase."

As used herein, the term "exonuclease" refers to an enzyme that cleaves nucleotides one at a time from the end of a polynucleotide chain. A hydrolyzing reaction occurs that breaks phosphodiester bonds at either the 3' or 5' ends. Its close relative is the endonuclease, which cleaves phosphodiester bonds in the middle of a polynucleotide chain. The related term "exonuclease activity" refers to the catalytic activity of an exonuclease.

As used herein, the term "strand-displacement activity" refers to the ability of a polymerase enzyme to displace a strand of an existing duplexed in the path of synthesis of a new strand. For example, in multiple-displacement amplification, the amplification reaction initiates when multiple primer hexamers anneal to the template. When DNA synthesis proceeds to the next starting site, the polymerase displaces the strand which was synthesized at that starting site and continues its strand elongation. For example, bacterial phage Φ29 DNA polymerase is a high proccessivity polymerase enzyme with strand displacement activity that can produce DNA 7 kb to 10 kb long. The reaction can be carried out at the moderate isothermal temperature condition of 30°C. It has been actively used in cell-free cloning, which is the enzymatic method of amplifying DNA *in vitro* without the need for cell culture and DNA extraction.

As used herein, the term "microfluidics" refers to technologies addressing the behavior, precise control and manipulation of fluids that are geometrically constrained to a sub-millimeter scale. It is a multidisciplinary field intersecting engineering, physics, chemistry, microtechnology and biotechnology, with practical applications to the design of systems in which such small volumes of fluids will be used.

As used herein, the terms "segment," "fragment," and "portion" when used in relation to polynucleotides or oligonucleotides of any kind, including the hairpin polynucleotides described herein, refer to a continuous sequence of nucleotide residues, which forms a subset of a larger sequence. For example, if a target nucleic acid is subjected to treatment with the transposase-hairpin polynucleotide complex, the oligonucleotides resulting from such treatment would represent segments or fragments of the starting target nucleic acid.

### III. Process Description

The hairpin polynucleotides used as starting materials in the methods described herein may be prepared synthetically, using either automation or conventional chemistry, for example by attaching a starting structure to beads and adding nucleotides thereto. Such methods are known to those skilled in the art. The hairpin oligonucleotides for use in the present invention may also be prepared by synthesizing segments or fragments thereof and then joining said segments or fragments into larger structures containing appropriate nucleotide sequences for use herein. Such segments or fragments may also be of natural origin, derived from microorganisms in nature or the result of cloning of sequences within selected organisms and utilizing selected vectors for the cloning process. Such segments or fragments may also be derived from natural vectors, such as plasmids, viruses, or the like.

The hairpin polynucleotides for use in the present invention may also be hybrids, or chimeras, containing some segments or sequences that are of natural origin as well as segments or sequences wholly synthetic in origin. The fact that a given segment or sequence is found in nature does not prevent it from being prepared synthetically in the laboratory for use herein.

An exemplary embodiment of a process of obtaining single-stranded circular nucleic acid from a double-stranded nucleic acid target is shown in Figure 1. A hairpin polynucleotide **10** is selected. The hairpin polynucleotide contains a transposase recognition sequence (not shown). The hairpin polynucleotide **10** is mixed with a transposase **12** to form a complex **14** which in this case, has two hairpin polynucleotides **10'** and **10"** bound to the transposase **12**.

In the next step of the process (shown in the middle portion of Figure 1), the complex **14** is then mixed with a double-stranded target nucleic acid **100** which, for the sake of clarity in this example, is shown with three regions; A-A' (left side), B-B' (right side) and C-C' (middle). The complex **14** binds to the target nucleic acid **100** and the enzymatic action of the transposase **12** of the complex **14** cleaves the target nucleic acid **100** within region C-C' to produce two nucleic acid fragments **102'** and **102"**. Nucleic acid fragment **102'** comprises duplex region A-A' linked to hairpin polynucleotide **10"** by the single-stranded gap E of region C'. Likewise, nucleic acid fragment **102"** comprises duplex region B-B" linked to hairpin polynucleotide **10'** by the single-stranded gap E' of region C.

In the next step of the process (shown at the bottom of Figure 1), the two fragments **102'** and **102"** are ligated together by addition of an enzyme with ligase activity such that the A-A' duplex region is linked to the B-B' duplex region. This produces an intermediate single-stranded nucleic acid **104** with two hairpin loops and two gaps E and E'. The two gaps E and E' are filled in by addition of a polymerase enzyme, thereby forming a circular single-stranded nucleic acid **106** having a duplex region and two opposing loops. In certain embodiments, it is useful to produce an open circle by melting the duplex region using known methods such as heat denaturation for example.

### IV. System Description

In another embodiment, there is disclosed herein a system for producing single-stranded circular nucleic acid. The system may be provided at either typical laboratory scale or at microfluidics scale where components of the system are provided on a chip and under the control of a computer. The system is provided with a plurality of reaction chambers where different steps in the process are carried out. Segregation of the process steps is expected to be necessary because certain reagents, buffers and additives needed for certain steps may be incompatible with other steps. For example, Mg²⁺ is needed for polymerase reactions but is thought to interfere with formation of stable complexes of Tn5 transposase with hairpin polynucleotides. Therefore, a separate chamber for formation of the complex may be advantageous.

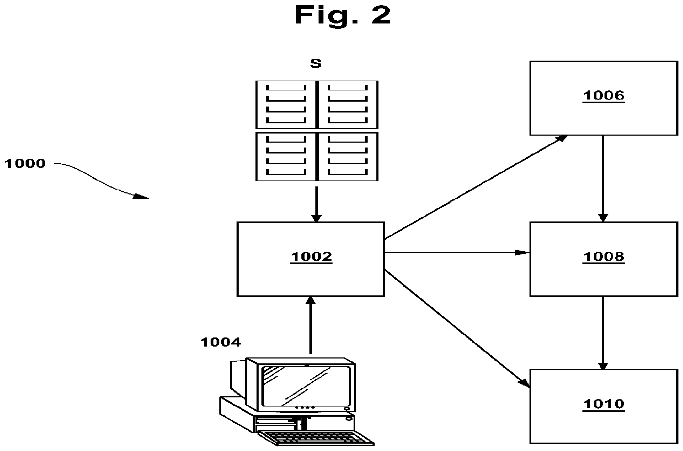

An example of this embodiment is shown in Figure 2 where it is seen that system **1000** includes a liquid handler **1002** under control of a computer **1004**. The liquid handler is configured for the transfer of liquids containing nucleic acid samples, intermediates and products obtained using the methods described above. System **1000** includes a first chamber **1006** for containing buffer components and reagents required for formation of a complex between a transposase and a plurality of hairpin polynucleotides. These components can also be charged into the first chamber **1006** by the liquid handler **1002** provided the liquid handler **1002** is in liquid handling communication with stock solutions of these components (not shown). If this is the case, the computer **1004** can be configured to control the communication between the stock solutions and the first chamber **1006** such that the composition of the solution used to form the complex between the transposase and the plurality of hairpin polynucleotides may be modified. This communication with stock solutions is also possible for preparing appropriate reaction conditions for the second chamber **1008** and the third chamber **1010**. It is also advantageous to have each of the chambers **1006, 1008**, and **1010**, under individual temperature control (not shown).

The liquid handler **1002** is configured to obtain a sample S comprising target nucleic acid and transfer the sample **S** to the second chamber **1008**. The liquid handler is also configured to transfer an aliquot of the solution containing the transposase-hairpin polynucleotide complex to the second chamber **1008**. In some embodiments, it may be advantageous to provide a purification module, such as a reversed phase chromatography column (not shown) at the exit point of one or more of the process chambers **1006**, **1008**, **1010** for removing buffer additives, salts and reagents in the event that it is found that these components interfere with the function of the next process chamber. For example, if the buffer used for forming the complex in the first chamber **1006** is incompatible with the process of fragmenting target nucleic acid in the second chamber **1008** a purification module can be provided at the outlet of the first chamber **1006** where buffer exchange can be carried out according to established methods known to those skilled in the art.

Once the target nucleic acid has been fragmented by the transposase-hairpin polynucleotide complex, it is transferred by the liquid handler **1002** to the third chamber **1010** which is provided with the reagents and enzymes needed to ligate the fragments and fill in the nucleobase segment gaps. The end result is the production of single-stranded circular nucleic acid which is useful for rolling circle amplification and rapid sequencing methods.

In certain embodiments, the hairpin polynucleotides are provided with tags which are specific for various sequencing platform technologies. When subjected to the methods described above, these tagged hairpin polynucleotides produce tagged libraries. Examples of such tags have been described for producing Nextera and Roche/454-compatible libraries for rapid sequencing platforms (Caruccio et al., Nextera™ Technology for NGS DNA Library Preparation: Simultaneous Fragmentation and Tagging by In Vitro Transposition Epicentre Forum 2009, 16-3, 4-6).

In some embodiments, a single-stranded circular nucleic acid library representing the genome of an organism or a virus is used as a template for obtaining bioagent identifying amplicons which provide base compositions that provide the means for rapid identification of the organism or virus through mass spectrometry according to methods described in patents, patent applications and scientific publications: U.S. patent numbers 7,108,974; 7,217,510; 7,226,739; 7,255,992; 7,312,036; 7,339,051; US patent publication numbers 2003/0027135; 2003/0167133; 2003/0167134; 2003/0175695; 2003/0175696; 2003/0175697; 2003/0187588; 2003/0187593; 2003/0190605; 2003/0225529; 2003/0228571; 2004/0110169; 2004/0117129; 2004/0121309; 2004/0121310; 2004/0121311; 2004/0121312; 2004/0121313; 2004/0121314; 2004/0121315; 2004/0121329; 2004/0121335; 2004/0121340; 2004/0122598; 2004/0122857; 2004/0161770; 2004/0185438; 2004/0202997; 2004/0209260; 2004/0219517; 2004/0253583; 2004/0253619; 2005/0027459; 2005/0123952; 2005/0130196 2005/0142581; 2005/0164215; 2005/0266397; 2005/0270191; 2006/0014154; 2006/0121520; 2006/0205040; 2006/0240412; 2006/0259249; 2006/0275749; 2006/0275788; 2007/0087336; 2007/0087337; 2007/0087338 2007/0087339; 2007/0087340; 2007/0087341; 2007/0184434; 2007/0218467; 2007/0218467; 2007/0218489; 2007/0224614; 2007/0238116; 2007/0243544; 2007/0248969; WO2002/070664; WO2003/001976; WO2003/100035; WO2004/009849; WO2004/052175; WO2004/053076; WO2004/053141; WO2004/053164; WO2004/060278; WO2004/093644; WO 2004/101809; WO2004/111187; WO2005/023083; WO2005/023986; WO2005/024046; WO2005/033271; WO2005/036369; WO2005/086634; WO2005/089128; WO2005/091971; WO2005/092059; WO2005/094421; WO2005/098047; WO2005/116263; WO2005/117270; WO2006/019784; WO2006/034294; WO2006/071241; WO2006/094238; WO2006/116127; WO2006/135400; WO2007/014045; WO2007/047778; WO2007/086904; WO2007/100397; WO2007/118222; Ecker et al., Ibis T5000: a universal biosensor approach for microbiology. Nat. Rev. Microbiol. 2008 Jun 3; Ecker et al., The Microbial Rosetta Stone Database: A compilation of global and emerging infectious microorganisms and bioterrorist threat agents. BMC Microbiology 2005, 5(1): 19; Ecker et al., The Ibis T5000 Universal Biosensor: An Automated Platform for Pathogen Identification and Strain Typing JALA, 2006, 6(11): 341-351; Ecker et al., The Microbial Rosetta Stone Database: A common structure for microbial biosecurity threat agents J. Forensic. Sci. 2005, 50(6): 1380-5; Ecker et al., Identification of Acinetobacter species and genotyping of Acinetobacter baumannii by multilocus PCR and mass spectrometry J. Clin. Microbiol. 2006, 44(8): 2921-32; Ecker et al., Rapid identification and strain-typing of respiratory pathogens for epidemic surveillance. Proc. Natl. Acad. Sci. U.S.A. 2005, 102(22): 8012-7; Wortmann et al., Genotypic evolution of Acinetobacter baumannii Strains in an outbreak associated with war trauma, Infect. Control Hosp. Epidemiol. 2008, 29(6):553-555; Hannis et al., High-resolution genotyping of Campylobacter species by use of PCR and high-throughput mass spectrometry J. Clin. Microbiol. 2008, 1220-5; Blyn et al., Rapid detection and molecular serotyping of adenovirus by use of PCR followed by electrospray ionization mass spectrometry J. Clin. Microbiol. 2008, 46(2): 644-51; Eshoo et al., Direct broad-range detection of alphaviruses in mosquito extracts Virology 2007, 368(2): 286-95; Sampath et al., Global surveillance of emerging Influenza virus genotypes by mass spectrometry. PLoS ONE, 2007, 2(5):e489; Sampath et al., Rapid identification of emerging infectious agents using PCR and electrospray ionization mass spectrometry. Ann. N.Y. Acad. Sci. 2007, 1102: 109-20; Hujer et al., Analysis of antibiotic resistance genes in multidrug-resistant Acinetobacter sp. isolates from military and civilian patients treated at the Walter Reed Army Medical Center Antimicrob. Agents Chemother. 2006, 50(12): 4114-23; Hall et al., Base composition analysis of human mitochondrial DNA using electrospray ionization mass spectrometry: a novel tool for the identification and differentiation of humans Anal. Biochem. 2005, 344(1): 53-69.; Sampath et al., Rapid identification of emerging pathogens: coronavirus Emerg. Infect. Dis. 2005, 11(3):373-9.; Jiang Y., Hofstadler S. A. A highly efficient and automated method of purifying and desalting PCR products for analysis by electrospray ionization mass spectrometry Anal. Biochem. 2003, 316: 50-57.; Jiang et al., Mitochondrial DNA mutation detection by electrospray mass spectrometry Clin. Chem. 2006, 53(2): 195-203.; Russell et al., Transmission dynamics and prospective environmental sampling of adenovirus in a military recruit setting. J. Infect. Dis. 2006, 194(7): 877-85.; Hofstadler et al., Detection of microbial agents using broad-range PCR with detection by mass spectrometry: The TIGER concept Encyclopedia of Rapid Microbiological Methods. 2006*.;* Hofstadler et al., Selective ion filtering by digital thresholding: A method to unwind complex ESI-mass spectra and eliminate signals from low molecular weight chemical noise Anal. Chem. 2006, 78(2): 372-378.; Hofstadler et al., TIGER: The Universal Biosensor Int. J. Mass Spectrom. 2005, 242(1): 23-41.; Van Ert et al., Mass spectrometry provides accurate characterization of two genetic marker types in Bacillus anthracis Biotechniques, 2004, 37(4): 642-4, 646, 648.; Sampath et al., Forum on Microbial Threats: Learning from SARS: Preparing for the Next Disease Outbreak-Workshop Summary (ed. Knobler SE, Mahmoud A, Lemon S.) The National Academies Press, Washington, D.C. 2004, 181-185.

While the present invention has been described with specificity in accordance with certain of its embodiments, the following examples serve only to illustrate the invention and are not intended to limit the same. In order that the invention disclosed herein may be more efficiently understood, examples are provided below. It should be understood that these examples are for illustrative purposes only and are not to be construed as limiting the invention in any manner.

### EXAMPLES

### Example 1: Optimization of Hairpin Polynucleotides for Formation of the Transposome Complex

Hairpin polynucleotides of various lengths with varying stem lengths, stem loops, blunt ends and staggered ends are prepared using standard oligonucleotide synthesis methods known to those skilled in the art. The hairpin polynucleotides contain transposase recognition sequences such as the end mosaic sequence of Tn5 transposase or the R1 and R2 sequences of MuA transposase. The positions of the transposase recognition sequence within the duplex region of the hairpin polynucleotides may be varied with the objective of optimizing the molecular recognition of the sequence by the transposase. Hairpin polynucleotides can be prepared with platform-specific sequencing tags as well. In these cases, the tagged hairpin polynucleotides are tested to ensure that the tags do not appreciably interfere with the process of complex formation.

Complex formation may be monitored using gel electrophoresis, or other binding assay method such as surface plasmon resonance spectroscopy for example.

In selecting appropriate characteristics for candidate hairpin polynucleotides, it may be advantageous to undertake molecular modeling studies for binding of hairpin polynucleotides to the selected transposase using published three-dimensional structures of the transposons and the newly designed hairpin polynucleotides. Such molecular modeling methods are known to those with skill in the art.

Buffer conditions are optimized for enhancing the process of formation of the complex. For example, reagents or stabilizers, such as Mg²⁺ for example, which are known to interfere with the process of complex formation, are excluded from the buffer used for complex formation.

### Example 2: Optimization of Incubation Times - to obtain optimal distribution of fragment lengths for individual applications.

For development of methods described herein, a standard target nucleic acid, for example, a simple viral test genome is selected, such as the genome of *H. influenzae* which contains 1.8 million base pairs.

The transposome-hairpin polynucleotide complex optimized according to Example 1 is then incubated with the test genome for varying periods of time in order to prepare fragments of optimal sizes. The optimal sizes chosen may depend upon the ultimate application of the single-stranded circular nucleic acid produced. The incubation temperature is carefully controlled to control the activity level of the transposome complex.

Conditions which may be appropriate for the fragmentation reaction were described for MuA transposase (Goldhaber-Gordon et al., J. Biol. Chem. 2002, 277, 7694-7702) and may be adapted to the fragmentation reaction as follows. The reactions are conducted in a 25 µl volume containing 25 mM Tris-HCl (pH 8 at room temperature), 140 mM NaCl, 10 mM MgCl₂, 1 mM dithiothreitol, 0.1 mg/ml bovine serum albumin, 15% glycerol, 12% dimethylsulfoxide, 0.1% Triton, 2 mM ATP, 250 ng of test DNA, and variable amounts of Mu DNA fragments and MuA. The MuA transposase is prepared by dilution of concentrated stock into 600 mM NaCl, 25 mM HEPES-KOH, 0.1 mM EDTA, 10% glycerol, and 1 mM dithiothreitol. The reactions are incubated at 30 °C. for 20-60 min.

### Example 3: Testing of Target Nucleic Acid Fragments for Preparation of Single-Stranded Circular Nucleic Acid Libraries

In this example, the fragments produced in Example 2 are treated with a ligase such as T4 ligase or *E. coli* ligase to ligate fragments together as indicated schematically in Figure 1. Advantageously, the filling in of the nucleobase segment gaps is also accomplished in the same reaction vessel by addition of a highly processive polymerase which lacks strand-displacement activity and 5'-3' exonuclease activity such as T4 or T7 polymerase. Reaction conditions are developed which are favorable to proper function of the polymerase and the ligase. If the filling of the gaps by the polymerase is found to be inefficient, the lack of a phosphorylated 5'-end in the nucleobase segment gap may be the cause of the inefficiency. A kinase can be added to ensure that the nucleobase segment gap is bordered by a phosphorylated 5'-end for proper polymerase function.

The single-stranded circular nucleic acid obtained from these tests can be purified by known methods and analyzed by gel electrophoresis, mass spectrometry, or various spectroscopic methods known to those skilled in the art. The single-stranded circular nucleic acid may also be tested as a template for amplification reactions such as rolling circle amplification or in rapid sequencing methods according to established procedures known to those skilled in the art.

Various modifications of the invention, in addition to those described herein, will be apparent to those skilled in the art from the foregoing description.

## Claims

1. A method for generating single stranded circular nucleic acid from a sample of target nucleic acid, said method comprising:
a) forming a complex comprising a transposase and a plurality of hairpin polynucleotides, each of said hairpin polynucleotides having a duplex region comprising a transposase recognition sequence;
b) mixing said complex with said target nucleic acid, thereby fragmenting said target nucleic acid and ligating said hairpin polynucleotides to said target nucleic acid, to form hairpin-linked nucleic acid fragments, each hairpin-linked nucleic acid fragment having a duplex target nucleic acid fragment region and a nucleobase segment gap between each target nucleic acid fragment region and its corresponding hairpin polynucleotide;
c) contacting said hairpin-linked fragments with a ligase, thereby ligating the duplex target nucleic acid fragment regions of said hairpin-linked fragments together to form single-stranded circular nucleic acid comprising a pair of opposing loops and an intervening duplex region, said duplex region comprising a pair of nucleobase segment gaps; and
d) contacting said single-single stranded circular nucleic acid of step c) with a polymerase and nucleotide triphosphates, thereby filling said nucleobase segment gaps.

2. A method for preparing a library of single-stranded circular nucleic acid which represents a genome of a virus or organism, said method comprising:
a) forming a complex comprising a transposase and a plurality of hairpin polynucleotides, each of said hairpin polynucleotides having a duplex region comprising a transposase recognition sequence;
b) mixing said complex with nucleic acid representing said genome, thereby fragmenting said nucleic acid and ligating said hairpin polynucleotides to said nucleic acid, to form hairpin-linked nucleic acid fragments, each hairpin-linked nucleic acid fragment having a duplex target nucleic acid fragment region and a nucleobase segment gap between each target nucleic acid fragment region and its corresponding hairpin polynucleotide;
c) contacting said hairpin-linked fragments with a ligase, thereby ligating the duplex target nucleic acid fragment regions of said hairpin-linked fragments together to form single-stranded circular nucleic acid comprising a pair of opposing loops and an intervening duplex region, said duplex region comprising a pair of nucleobase segment gaps; and
d) contacting said single-single stranded circular nucleic acid of step c) with a polymerase and nucleotide triphosphates, thereby filling said nucleobase segment gaps.

3. The method of claim 1 or 2 further comprising:
heating said single-stranded circular nucleic acid to denature said intervening duplex region; and/or
mixing said hairpin-linked fragments with a kinase before or during step d) to phosphorylate any non-phosphorylated 5' ends prior to filling said nucleobase segment gaps.

4. The method of claim 1 or 2 wherein said transposase:
catalyzes random integration of said hairpin polynucleotide into said target nucleic acid; and/or
is MuA transposase or Tn5 transposase;
is Tn5 transposase and said transposase recognition sequence is the 19-base pair mosaic end sequence of the Tn5 transposon; or
is MuA transposase and said transposase recognition sequence is the R1 or the R2 region of the MuA transposon.

5. The method of claim 1 or 2 wherein:
said polymerase lacks 5'-3' exonuclease activity;
said polymerase lacks strand-displacement activity; and/or
said polymerase is T4 polymerase or T7 polymerase;
said ligase is T4 ligase; and/or
said hairpin polynucleotides comprise sequencing tags.

## Patentansprüche

1. Verfahren zur Herstellung von einzelsträngiger zirkulärer Nukleinsäure aus einer Probe von Zielnukleinsäure, wobei das Verfahren Folgendes umfasst:
a) Bilden eines Komplexes, der eine Transposase und eine Mehrzahl an Haarnadelschleifen-Polynukleotiden umfasst, wobei jedes der Haarnadelschleifen-Polynukleotide eine Duplexregion aufweist, die eine Transposase-Erkennungssequenz umfasst;
b) Mischen des Komplexes mit der Zielnukleinsäure, wodurch die Zielnukleinsäure fragmentiert wird, und Ligieren der Haarnadelschleifen-Polynukleotide an die Zielnukleinsäure unter Bildung von Haarnadelschleifen-verknüpften Nukleinsäurefragmenten, wobei jedes Haarnadelschleifen-verknüpfte Nukleinsäurefragment eine Duplex-Zielnukleinsäurefragmentregion und eine Nukleobasensegmentlücke zwischen jeder Zielnukleinsäurefragmentregion und seinem entsprechenden Haarnadelschleifen-Polynukleotid besitzt;
c) Inkontaktbringen der Haarnadelschleifen-verknüpften Fragmente mit einer Ligase, wodurch die Duplex-Zielnukleinsäurefragmentregionen der Haarnadelschleifen-verknüpften Fragmente miteinander ligiert werden unter Bildung von einzelsträngiger zirkulärer Nukleinsäure, die ein Paar gegenüberliegender Schleifen und eine dazwischenliegende Duplexregion umfasst, wobei die Duplexregion ein Paar von Nukleobasensegmentlücken umfasst, und
d) Inkontaktbringen der einzelsträngigen zirkulären Nukleinsäure von Schritt c) mit einer Polymerase und Nukleotidtriphosphaten, wodurch die Nukleobasensegmentlücken aufgefüllt werden.

2. Verfahren zur Herstellung einer Bank von einzelsträngiger zirkulärer Nukleinsäure, die ein Genom eines Virus oder Organismus darstellt, wobei das Verfahren Folgendes umfasst:
a) Bilden eines Komplexes, der eine Transposase und eine Mehrzahl an Haarnadelschleifen-Polynukleotiden umfasst, wobei jedes der Haarnadelschleifen-Polynukleotide eine Duplexregion aufweist, die eine Transposase-Erkennungssequenz umfasst;
b) Mischen des Komplexes mit der Nukleinsäure, die das Genom darstellt, wodurch die Nukleinsäure fragmentiert wird, und Ligieren der Haarnadelschleifen-Polynukleotide an die Nukleinsäure unter Bildung von Haarnadelschleifen-verknüpften Nukleinsäurefragmenten, wobei jedes Haarnadelschleifen-verknüpfte Nukleinsäurefragment eine Duplex-Zielnukleinsäurefragmentregion und eine Nukleobasensegmentlücke zwischen jeder Zielnukleinsäurefragmentregion und seinem entsprechenden Haarnadelschleifen-Polynukleotid besitzt;
c) Inkontaktbringen der Haarnadelschleifen-verknüpften Fragmente mit einer Ligase, wodurch die Duplex-Zielnukleinsäurefragmentregionen der Haarnadelschleifen-verknüpften Fragmente miteinander ligiert werden unter Bildung von einzelsträngiger zirkulärer Nukleinsäure, die ein Paar gegenüberliegender Schleifen und eine dazwischenliegende Duplexregion umfasst, wobei die Duplexregion ein Paar von Nukleobasensegmentlücken umfasst, und
d) Inkontaktbringen der einzelsträngigen zirkulären Nukleinsäure von Schritt c) mit einer Polymerase und Nukleotidtriphosphaten, wodurch die Nukleobasensegmentlücken aufgefüllt werden.

3. Verfahren nach Anspruch 1 oder 2, das weiterhin Folgendes umfasst:
Erhitzen der einzelsträngigen zirkulären Nukleinsäure, um die dazwischenliegende Duplexregion zu denaturieren, und/oder
Mischen der Haarnadelschleifen-verknüpften Fragmente mit einer Kinase vor oder während Schritt d), um sämtliche nicht-phosphorylierten 5'-Enden vor dem Auffüllen der Nukleobasensegmentlücken zu phosphorylieren.

4. Verfahren nach Anspruch 1 oder 2, wobei die Transposase:
die zufallsgemäße Integration des Haarnadelschleifen-Polynukleotids in die Zielnukleinsäure katalysiert und/oder
MuA-Transposase oder Tn5-Transposase ist,
Tn5-Transposase ist und die Transposase-Erkennungssequenz die 19 Basenpaare lange "Mosaic End"-Sequenz des Tn5-Transposons ist oder
MuA-Transposase ist und die Transposase-Erkennungssequenz die R1- oder R2-Region des MuA-Transposons ist.

5. Verfahren nach Anspruch 1 oder 2, wobei:
der Polymerase 5'-3'-Exonuklease-Aktivität fehlt,
der Polymerase Strangverdrängungsaktivität fehlt und/oder
die Polymerase T4-Polymerase oder T7-Polymerase ist,
die Ligase T4-Ligase ist und/oder
die Haarnadelschleifen-Polynukleotide Sequenzierungs-Tags umfassen.

## Revendications

1. Procédé de génération d'acide nucléique simple brin circulaire à partir d'un échantillon d'acide nucléique cible, ledit procédé comprenant :
a) la formation d'un complexe comprenant une transposase et une pluralité de polynucléotides en épingle à cheveux, chacun desdits polynucléotides en épingle à cheveux ayant une région de double hélice comprenant une séquence de reconnaissance de transposase ;
b) le mélange dudit complexe avec ledit acide nucléique cible, de manière à fragmenter ledit acide nucléique cible et ligaturer lesdits polynucléotides en épingle à cheveux avec ledit acide nucléique cible, pour former des fragments d'acide nucléique liés en épingle à cheveux, chaque fragment d'acide nucléique lié en épingle à cheveux ayant une région de fragment d'acide nucléique cible en double hélice et une brèche de segment de bases azotées entre chaque région de fragment d'acide nucléique cible et son polynucléotide en épingle à cheveux correspondant ;
c) la mise en contact desdits fragments liés en épingle à cheveux avec une ligase, de manière à ligaturer les régions de fragment d'acide nucléique cible en double hélice desdits fragments liés en épingle à cheveux conjointement pour former un acide nucléique circulaire simple brin comprenant une paire de boucles opposées et une région de double hélice intercalée, ladite région de double hélice comprenant une paire de brèches de segment de bases azotées ; et
d) la mise en contact dudit acide nucléique circulaire simple brin de l'étape c) avec une polymérase et des nucléotide triphosphates, de manière à remplir les brèches de segment de bases azotées.

2. Procédé de préparation d'une banque d'acide nucléique circulaire simple brin qui représente un génome d'un virus ou organisme, ledit procédé comprenant :
a) la formation d'un complexe comprenant une transposase et une pluralité de polynucléotides en épingle à cheveux, chacun desdits polynucléotides en épingle à cheveux ayant une région de double hélice comprenant une séquence de reconnaissance de transposase ;
b) le mélange dudit complexe avec un acide nucléique représentant ledit génome, de manière à fragmenter ledit acide nucléique et ligaturer lesdits polynucléotides en épingle à cheveux avec ledit acide nucléique cible, pour former des fragments d'acide nucléique liés en épingle à cheveux, chaque fragment d'acide nucléique lié en épingle à cheveux ayant une région de fragment d'acide nucléique cible en double hélice et une brèche de segment de bases azotées entre chaque région de fragment d'acide nucléique cible et son polynucléotide en épingle à cheveux correspondant ;
c) la mise en contact desdits fragments liés en épingle à cheveux avec une ligase, de manière à ligaturer les régions de fragment d'acide nucléique cible en double hélice desdits fragments liés en épingle à cheveux conjointement de manière à former un acide nucléique circulaire simple brin comprenant une paire de boucles opposées et une région de double hélice intercalée, ladite région de double hélice comprenant une paire de brèches de segment de bases azotées ; et
d) la mise en contact dudit acide nucléique circulaire simple brin de l'étape c) avec une polymérase et des nucléotide triphosphates, de manière à remplir lesdites brèches de segment de bases azotées.

3. Procédé de la revendication 1 ou 2 comprenant en outre :
le chauffage dudit acide nucléique circulaire simple brin pour dénaturer ladite région de double hélice intercalée ; et/ou
le mélange desdits fragments liés en épingle à cheveux avec une kinase avant ou pendant l'étape d) pour phosphoryler les extrémités 5' éventuellement non phosphorylées avant le remplissage desdites brèches de segment de bases azotées.

4. Procédé de la revendication 1 ou 2 dans lequel ladite transposase :
catalyse l'intégration aléatoire dudit polynucléotide en épingle à cheveux dans ledit acide nucléique cible ; et/ou
est la transposase MuA ou la transposase Tn5 ;
est la transposase Tn5 et ladite séquence de reconnaissance de transposase est la séquence terminale mosaïque de 19 paires de bases du transposon Tn5 ; ou
est la transposase MuA et ladite séquence de reconnaissance de transposase est la région R1 ou R2 du transposon MuA.

5. Procédé de la revendication 1 ou 2 dans lequel :
ladite polymérase ne comporte pas d'activité 5'-3'-exonucléase ;
ladite polymérase ne comporte pas d'activité de déplacement de brin ; et/ou
ladite polymérase est la polymérase T4 ou la polymérase T7 ;
ladite ligase est la ligase T4 ; et/ou
lesdits polynucléotides en épingle à cheveux comprennent des étiquettes de séquençage.
